# EUROPEAN PATENT APPLICATION

(11) **EP 4 699 524 A1**
(43) Date of publication of application: **25.02.2026**
(21) Application number: 24780303.4
(22) Date of filing: 26.03.2024
(51) Int. Cl.: A61B 5/02, A61B 5/00, A61B 5/01, A61B 5/11, A61B 10/00

(54) **WEARABLE DEVICE AND HEART FAILURE DETECTION SYSTEM**

(30) Priority: 28.03.2023 JP 2023051480
(71) Applicant: The University of Osaka, Osaka 565-0871 (JP)
(72) Inventor: MASUDA, Hirotada, Suita-shi, Osaka 565-0871 (JP); MIYAGAWA, Shigeru, Suita-shi, Osaka 565-0871 (JP); YAGI, Masakazu, Suita-shi, Osaka 565-0871 (JP)
(74) Representative: EIP
(86) International application number: PCT/JP2024/011938
(87) International publication number: WO 2024/204193

(57) **Abstract**

An inexpensive means is provided which monitors a heart condition of a subject such as a patient in a less invasive manner than conventional methods without significantly compromising the reliability of acquiring heart sounds. In a wearable device 2 that is worn on a wrist of a subject and used, a sound collection portion including rubber 623 for acquiring heart sounds of the subject is disposed on an upper surface 201, and a user interface unit 65 operated by the subject is disposed on a side surface 204 along the longitudinal direction of an arm of the subject when the wearable device 2 is worn on the wrist of the subject.

## Description

### Technical Field

The present invention relates to a wearable device for collecting heart sounds.

### Background Art

In recent years, the rate of chronic heart failure in Japan has continued to rise as the population has aged. As of 2016, approximately one million people in Japan develop chronic heart failure annually, of which about 100,000 patients need hospitalization for treatment. Even after completing treatment and being discharged, about 40% of patients experience a recurrence of severe symptoms within a year, which results them being readmitted to hospital. The estimated nationwide medical costs related to the readmissions are 48 billion yen.

Ensuring reliable monitoring of a patient's heart condition after discharge, detecting recurrence at an early stage, and intervening promptly are key to avoiding hospital readmission.

Various useful indicators for monitoring are being researched worldwide. Among existing monitoring methods, CardioMEMS provided by Abbott Laboratories, which directly monitors pulmonary artery pressure, has been commercialized as an effective means.

In addition, the following technologies have been proposed. The heart monitoring device described in Patent Literature 1 includes an enclosure with various sensors and components. The enclosure has a first groove on the top surface and the first groove is sized to fit phalanges of the subject's right hand. When the heart monitoring device is held with the corresponding phalanges pressed against the first groove, the heart monitoring device is oriented properly to record the subject's heart activity. The heart monitoring device includes a plurality of electrodes configured to create one or more electric circuits through a human heart. The plurality of electrodes includes a right thumb electrode disposed on a side surface of the enclosure for coupling to the subject's right thumb, and an upper chest electrode as well as a lower chest electrode disposed on a bottom surface of the enclosure for coupling to the subject's chest. The heart monitoring device also has a plurality of pulse oximeters disposed in the first groove and configured to measure the blood oxygen level of the corresponding phalanges.

The heart rate monitor described in Patent Literature 2 is hung around the subject's neck like a pendant to measure the heart rate near the chest.

The heart rate monitor described in Patent Literature 3 is a wristwatch-type heart rate monitor with a microphone attached to the wristband.

### Citation List

### Non-Patent Literature

Non-Patent Literature 1: CardioMEMS HF System, https://www.cardiovascular.abbott/us/en/hcp/products/heart-failure/pulmonary-pressure-monitors/cardiomems/about.html, searched on March 11, 2023

### Patent Literature

Patent Literature 1: Japanese Unexamined Patent Publication No. 2021-501029
Patent Literature 2: Japanese Unexamined Patent Publication No. H2-074232
Patent Literature 3: Japanese Unexamined Patent Publication No. H5-029686

### Summary of Invention

### Technical Problem

However, the conventional monitoring means described in Non-Patent Literature 1 involves implanting a device in pulmonary artery of a patient. In short, it is invasive. Consequently, it places a burden on the patient's body and increases the workload for physicians. Further, the process is costly and measurements can only be made using a special bed sensor. Additionally, the device or the heart rate monitor described in Patent Literature 1 is less reliable in acquiring heart sounds.

The present invention has been achieved in light of such problems, and therefore, an object of the present invention is to provide an inexpensive means for monitoring a heart condition of a subject such as a patient in a less invasive manner than conventional methods without significantly compromising the reliability of acquiring heart sounds.

### Solution to Problem

A wearable device according to an aspect of the present invention has a sound collection portion that is provided on an upper surface or a lower surface of a body of the wearable device and is configured to acquire heart sounds of a subject, and an operation portion that is provided on a side surface of the body along a longitudinal direction of an arm of the subject when the wearable device is worn on a wrist of the subject and is configured to perform, by the subject, operation for acquiring the heart sounds.

Preferably, the sound collection portion includes rubber and a microphone having a piezoelectric element, and a surface of the rubber forms a part of the upper surface, and the microphone is disposed in contact with a back surface of the rubber.

### Advantageous Effects of Invention

According to the present invention, it is possible to provide an inexpensive means for monitoring a heart condition of a subject such as a patient in a less invasive manner than conventional methods.

### Brief Description of Drawings

FIG. 1 is a diagram illustrating an example of the overall configuration of a heart failure detection system.
FIG. 2 is a perspective view of a wearable device.
FIG. 3 is a diagram illustrating an example of how a wearable device 2 is worn in normal circumstances.
FIG. 4 is a diagram illustrating an example of how the wearable device 2 is worn when collecting heart sounds.
FIG. 5 is an exploded view of a wearable device.
FIG. 6 is a diagram illustrating an example of an upper surface, a lower surface, and three side surfaces of a wearable device, using third angle projection.
FIG. 7 is a diagram illustrating an example of how hardware is implemented on a main board.
FIG. 8 is a diagram illustrating an example of the configuration of an observation support program.
FIG. 9 is a diagram illustrating an example of control on three light emitters for each state.
FIG. 10 is a diagram illustrating an example of a waveform of heart sounds.
FIG. 11 is a flowchart illustrating an example of the overall processing flow by an observation support program.
FIG. 12 is a flowchart illustrating an example of the overall processing flow by an observation support program.
FIG. 13 is a diagram illustrating a first modification to the wearable device.
FIG. 14 is a diagram illustrating a second modification to the wearable device.
FIG. 15 is a diagram illustrating a modification to the arrangement of three contacts.
FIG. 16 is a diagram illustrating an example of the appearance of a wearable device.
FIG. 17 is a diagram illustrating an example of the appearance of an upper surface side and a lower surface side of a device body.
FIG. 18 is a diagram illustrating an example of the appearance of a side surface side and a lower surface side of a wearable device.
FIG. 19 is a front view and a perspective view of a coupling member.
FIG. 20 is a diagram illustrating an example of a female part and a male part.
FIG. 21 is a diagram illustrating an example of the appearance of an upper surface side and a lower surface side of a wearable device.

### Description of Embodiments

### (1. Overall configuration of heart failure detection system 1)

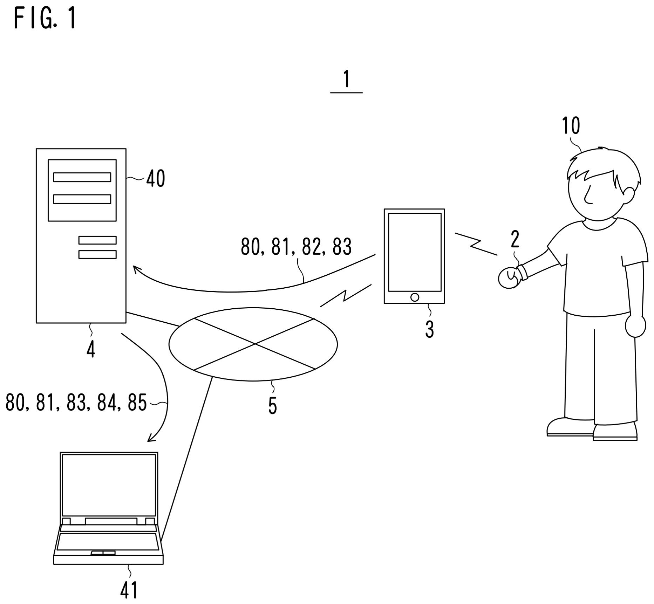

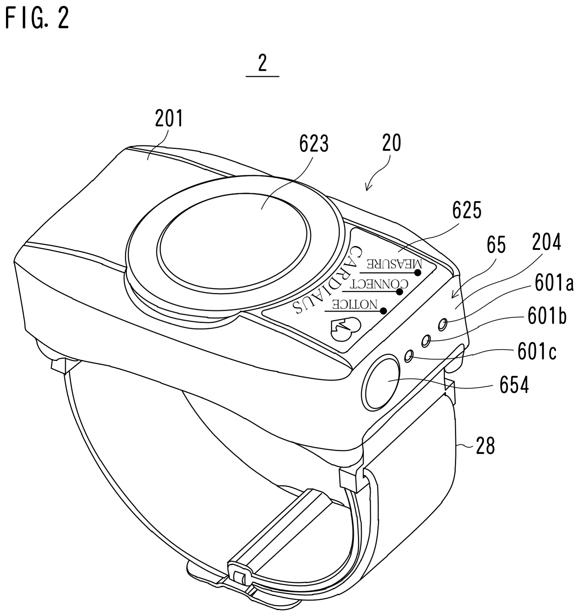

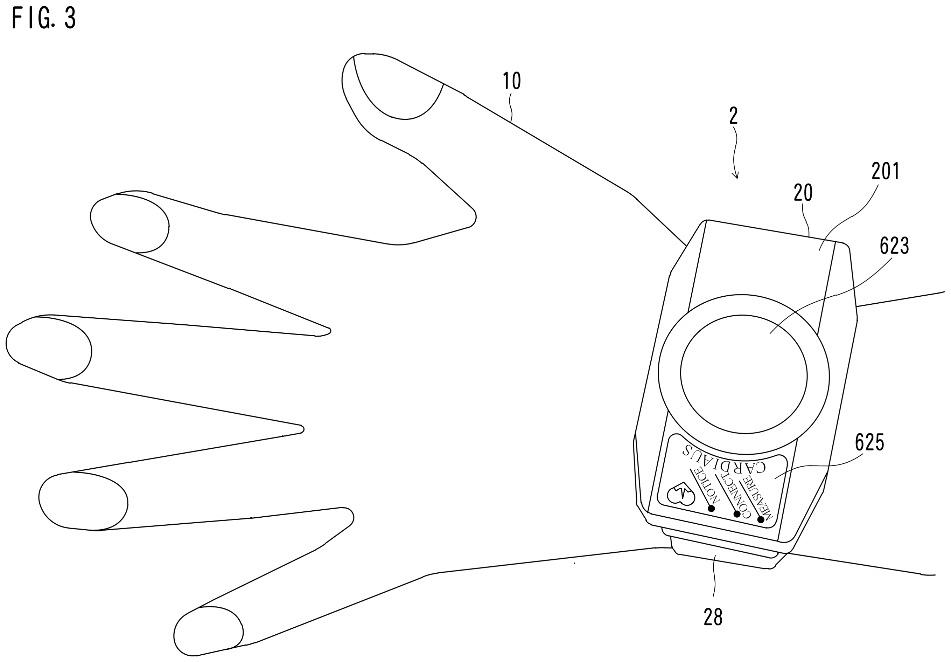

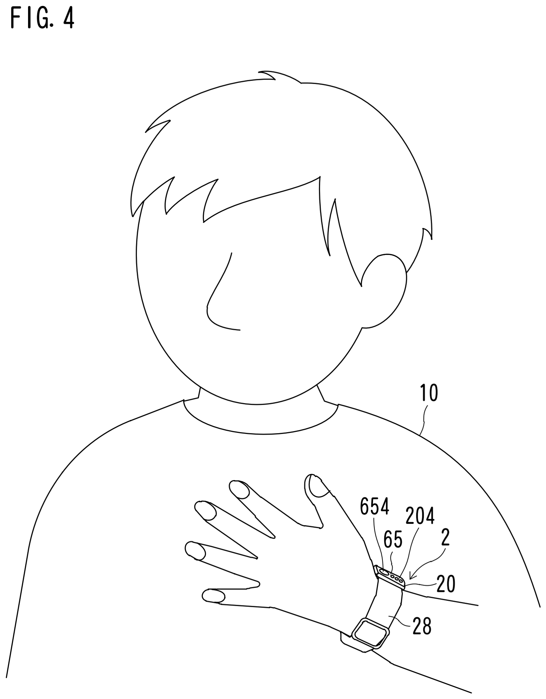

FIG. 1 is a diagram illustrating an example of the overall configuration of the heart failure detection system 1. FIG. 2 is a perspective view of a wearable device 2. FIG. 3 is a diagram illustrating an example of how the wearable device 2 is worn in normal circumstances. FIG. 4 is a diagram illustrating an example of how the wearable device 2 is worn when collecting heart sounds.

The heart failure detection system 1 is a system for detecting heart failure in a subject 10. As illustrated in FIG. 1, the heart failure detection system 1 includes the wearable device 2, a relay device 3, a heart failure detection server 40, a medical personnel terminal 41, and a communication line 5.

The wearable device 2 is a wristwatch-type device for recording heart sounds of the subject 10, and includes a device body 20 and a wristband 28 as illustrated in FIG. 2.

The subject 10 wears the wearable device 2 on his/her wrist throughout the day, in principle. The subject 10 can wear the wearable device 2 on either the left or right wrist; however, it is desirable to wear the wearable device 2 so that a user interface unit 65 and a display sticker 625 are located on the thumb side when the device body 20 is on the palmer side.

When heart sounds are not recorded, that is, normally, the subject 10 wears the wearable device 2 with the device body 20 located on the dorsal side as illustrated in FIG. 3. On the other hand, in order to record heart sounds, the subject 10 rotates the device body 20, while still wearing the wearable device 2, around toward the palmer side and presses the device body 20 against the chest near the heart of the subject 10, as illustrated in FIG. 4. Incidentally, the subject 10 can wear the wearable device 2 so that the device body 20 is located on the palmer side even when heart sounds are not recorded.

Referring back to FIG. 1, the wearable device 2 and the relay device 3 communicate with each other via short-range wireless communication such as Bluetooth.

The relay device 3 receives, from the wearable device 2, heart sounds recorded by the wearable device 2 and so on, and transfers the same via the communication line 5 to the heart failure detection server 40. Examples of the relay device 3 include a portable device having a function for short-range wireless communication and a function for internet protocol (IP) communication, e.g., a smartphone and a tablet computer. The communication line 5 is an IP communication network such as an Internet line or a LAN line.

The heart failure detection server 40 analyzes the heart sounds transferred from the relay device 3 to detect heart failure in the subject 10. Examples of the heart failure detection server 40 include a so-called server machine and cloud server.

The medical personnel terminal 41 is a terminal used by medical personnel in charge of the subject 10. Examples of the medical personnel terminal 41 include a personal computer, a smartphone, and a tablet computer.

### (2. Hardware configuration of wearable device 2)

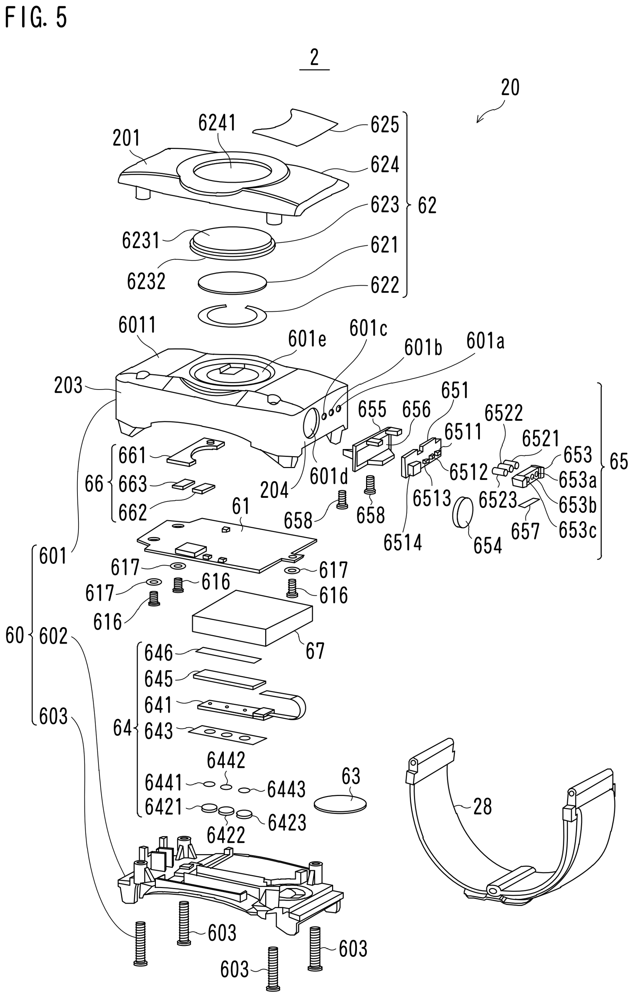

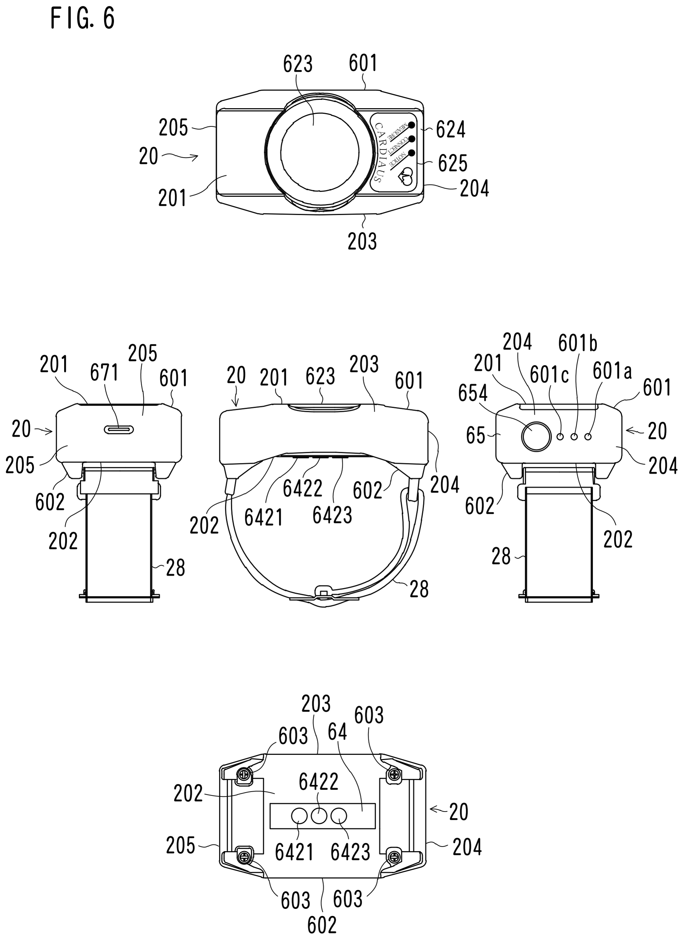

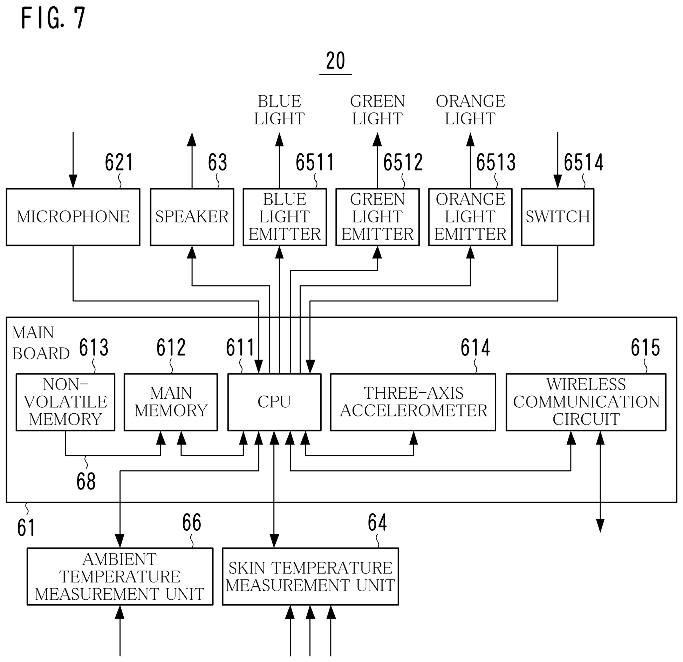

FIG. 5 is an exploded view of the wearable device 2. FIG. 6 is a diagram illustrating an example of an upper surface 201, a lower surface 202, and three side surfaces 203 to 205 of the wearable device 2, using third angle projection. FIG. 7 is a diagram illustrating an example of how hardware is implemented on a main board 61.

In FIG. 6, a front view is illustrated in the center, a top view and a bottom view are illustrated above and below, respectively, and a left-side view and a right-side view are illustrated on the left and right of the front view, respectively. Incidentally, the wristband 28 is omitted from the bottom view.

As illustrated in FIG. 6, FIG. 5, or FIG. 7, the device body 20 includes a cabinet 60, the main board 61, a sound collection unit 62, a speaker 63, a skin temperature measurement unit 64, the user interface unit 65, an ambient temperature measurement unit 66, and a secondary battery 67.

The cabinet 60 is a casing for the device body 20, and includes a top cabinet 601, a bottom cabinet 602, and four screws 603 as illustrated in FIG. 5. The bottom cabinet 602 is fixed to the top cabinet 601 with the screws 603.

The main board 61, the speaker 63, the skin temperature measurement unit 64, the user interface unit 65, the ambient temperature measurement unit 66, and the secondary battery 67 are housed in the space between the top cabinet 601 and the bottom cabinet 602. However, as described later, some components slightly protrude beyond the top cabinet 601 or the bottom cabinet 602.

The main board 61 is fixed to the top cabinet 601 with three sets of screws 616 and washers 617.

The main board 61 is equipped with a central processing unit (CPU) 611, a main memory 612, a non-volatile memory 613, a three-axis accelerometer 614, and a wireless communication circuit 615 illustrated in FIG. 7.

An observation support program 68 is installed in the non-volatile memory 613. The observation support program 68 is loaded into the main memory 612 and executed by the CPU 611.

The three-axis accelerometer 614 is used to measure the activity level of the subject 10. The wireless communication circuit 615 is used to perform wireless communication with the wearable device 2.

As illustrated in FIG. 5, the sound collection unit 62 includes a microphone 621, a double-sided tape 622, rubber 623, a lid 624, and the display sticker 625.

The microphone 621 is fixed to a recess 601e provided at the center of an upper surface 6011 of the top cabinet 601 with the double-sided tape 622. Examples of the microphone 621 include a circular piezoelectric element with a diameter of approximately 20 millimeters. The piezoelectric element is sold under names such as a "piezoelectric sheet", a "piezoelectric microphone", a "piezoelectric film", or a "piezoelectric buzzer". Incidentally, a hole is provided near the center of the bottom surface of the recess 601e, and an electric wire connecting the microphone 621 and the main board 61 passes through the hole.

The rubber 623 is a circular and thin rubber film, and is provided on a part corresponding to a dial of the wristwatch. More specifically, a lower surface 6232 is placed to contact and cover the microphone 621. Further, the rubber 623 has an upper surface 6231 that is exposed on the upper surface 6011 of the top cabinet 601, and the upper surface 6231 is pressed against the chest of the subject 10 when heart sounds are recorded. Thereby, the rubber 623 serves to convey the heart sounds to the microphone 621. The upper surface 6231 of the rubber 623 has an end (periphery) cut out, and a step is formed to fit into a hole 6241 of the lid 624.

The microphone 621 and the rubber 623 are attached to an upper part of the top cabinet 601 and closed by the lid 624. The lid 624 is provided with the hole 6241 into which an upper part of the rubber 623 is fitted. As a result, the upper part of the rubber 623 protrudes slightly outward from the lid 624.

As illustrated in FIG. 6, on the display sticker 625, the main role of each of blue, green, and orange colors emitted by light emitters of the user interface unit 65 is printed, and the display sticker 625 is bonded to the upper surface of the lid 624 on the user interface unit 65 side.

The speaker 63 outputs sounds such as beeps. Examples of the speaker 63 include a piezoelectric element.

The skin temperature measurement unit 64 includes a skin temperature measurement board 641, a first contact 6421, a second contact 6422, a third contact 6423, a Kapton tape 643, contact tapes 6441 to 6443, a spacer 645, and a battery tape 646, and measures the temperature at each of three points on the skin of the wrist of the subject 10. Incidentally, "Kapton" is a registered trademark of DuPont de Nemours, Inc.

The first contact 6421, the second contact 6422, and the third contact 6423 are probes that come into contact with the skin, and are coupled to the skin temperature measurement board 641 via the Kapton tape 643 and the contact tapes 6441 to 6443.

The bottom surface of the bottom cabinet 602 has three holes arranged on a straight line perpendicular to the longitudinal direction of an arm of the subject 10 when being worn by the subject 10. The first contact 6421, the second contact 6422, the third contact 6423, and the holes each have a diameter of approximately 6 millimeters, and the distance between two adjacent holes is approximately 2 millimeters. The same is applied to the distance between two adjacent contacts. Thus, the distance between the centers of two adjacent contacts is approximately 8 millimeters. The first contact 6421, the second contact 6422, and the third contact 6423 are fitted into the holes and protrude slightly beyond the bottom cabinet 602.

The skin temperature measurement board 641 is fixed to the secondary battery 67 via the spacer 645 and the battery tape 646.

The skin temperature measurement board 641 is equipped with a sensor and a circuit for measuring the temperature of the heat transferred from the first contact 6421, the second contact 6422, and the third contact 6423. This allows the temperature at each of the three points on the skin of the wrist of the subject 10 to be measured. The measured values are provided to the CPU 611.

The user interface unit 65 includes a side key board 651, a blue light guide pin 6521, a green light guide pin 6522, an orange light guide pin 6523, a light-shielding block 653, a start key 654, a side key holder 655, a side key board tape 656, a side key holder tape 657, and two screws 658.

The side key board 651 is equipped with a blue light emitter 6511, a green light emitter 6512, an orange light emitter 6513, a switch 6514, and so on. The blue light emitter 6511, the green light emitter 6512, and the orange light emitter 6513 are light emitting diodes (LEDs) that emit blue, green, and orange light, respectively.

The blue light emitter 6511 conveys a state regarding acquisition of heart sounds and body temperature. The green light emitter 6512 conveys a state regarding communication with the relay device 3. The orange light emitter 6513 conveys a state regarding charging.

The start key 654 is a cylindrical button, and the back surface thereof can come into contact with an operation unit of the switch 6514. Meanwhile, the top cabinet 601 has two side surfaces 204 and 205 perpendicular to the periphery of the wristband 28, and the side surface 204 of the two side surfaces 204 and 205 is provided with holes 601a to 601c and a hole 601d. The start key 654 is fitted into the hole 601d from the inside, and the surface of the start key 654 slightly protrudes outward beyond the top cabinet 601. The side surface 204 is sometimes referred to as an "operation surface 204".

When the subject 10 presses the start key 654, the operation unit of the switch 6514 is pressed in conjunction, and a predetermined signal is sent to the CPU 611 of the main board 61.

The blue light guide pin 6521, the green light guide pin 6522, and the orange light guide pin 6523 are light-transmitting pins, and are provided to come into contact with the blue light emitter 6511, the green light emitter 6512, and the orange light emitter 6513, respectively.

The light-shielding block 653 is a block configured to shield light. The light-shielding block 653 has three holes 653a, 653b, and 653c, into which the blue light guide pin 6521, the green light guide pin 6522, and the orange light guide pin 6523 are fitted and held, respectively. Ends of the blue light guide pin 6521, the green light guide pin 6522, and the orange light guide pin 6523 are visible from the holes 601a, 601b, and 601c of the top cabinet 601, respectively.

With this configuration, when the blue light emitter 6511, the green light emitter 6512, and the orange light emitter 6513 emit light, blue, green, and orange light passes through the blue light guide pin 6521, the green light guide pin 6522, and the orange light guide pin 6523 respectively to reach the outside of the device body 20. This allows the subject 10 to confirm the light of each color.

The side key board 651 is fitted into the side key holder 655. The side key board tape 656 firmly secures the side key board 651 to the side key holder 655. The side key holder tape 657 and the screws 658 secure the side key holder 655 to the top cabinet 601.

The ambient temperature measurement unit 66 includes an ambient temperature measurement board 661, a spacer 662, and a spacer tape 663.

The ambient temperature measurement board 661 is equipped with a sensor and a circuit for measuring the temperature around the wearable device 2. When the subject 10 is inside a room, the temperature of the room (room temperature) is measured. When the subject 10 is outside, the external temperature is measured.

The ambient temperature measurement board 661 is provided on the main board 61 via the spacer 662. The spacer tape 663 secures the spacer 662 to the ambient temperature measurement board 661.

The secondary battery 67 supplies power to each unit of the device body 20. Further, the secondary battery 67 is connected to a universal serial bus (USB) power source and charged via a USB cable or the like inserted into a USB connector 671 (see FIG. 6) of the side surface 205.

Further, the device body 20 is provided with a circuit for controlling the supply of power from the secondary battery 67 to the individual units, a circuit for controlling charging to the secondary battery 67, and a circuit for exchanging signals between the individual units.

The wristband 28 is wrapped around the wrist of the subject 10 to secure the device body 20 to the wrist of the subject 10. The wristband 28 may be a commercially available wristwatch band. As described above, the position of the device body 20 is changed in order to record heart sounds, so it is desirable to use an elastic band, such as a rubber band or a nylon band, for the wristband 28, which allows the device body 20 to move easily. Alternatively, a braided band is desirably used. In addition, the wristband 28 may be a leather band, a steel band, a silicone band, a synthetic resin band, and a canvas bands.

### (3. Software configuration of wearable device 2)

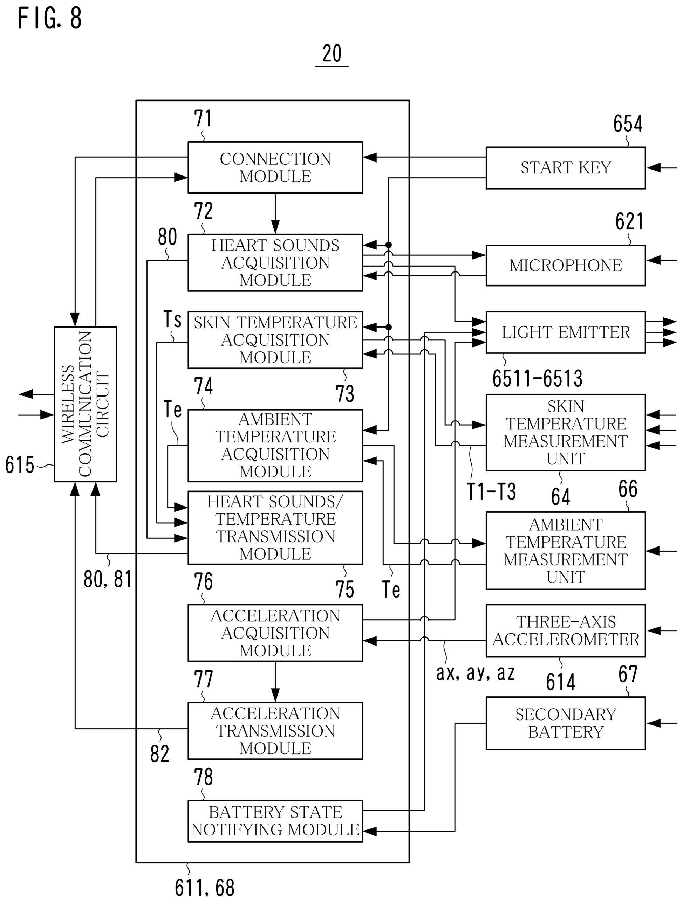

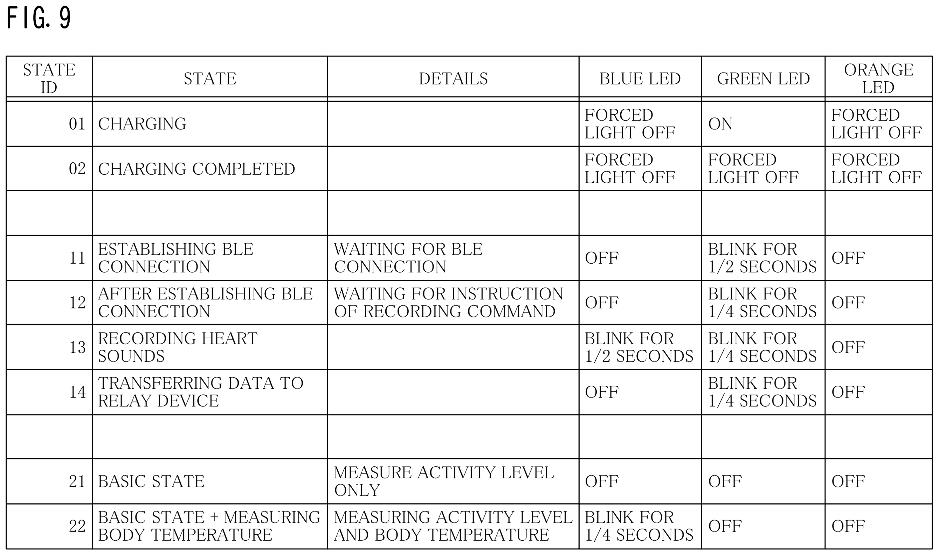

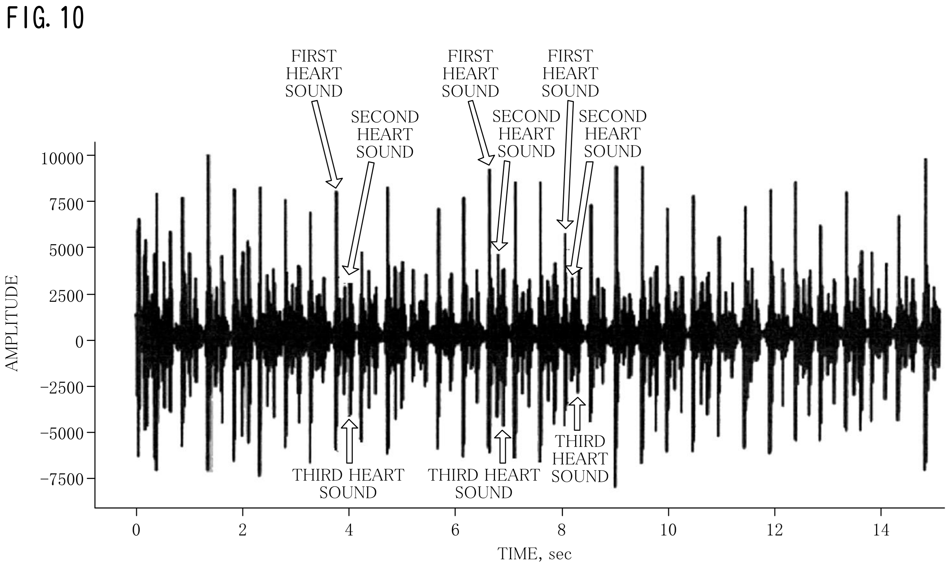

FIG. 8 is a diagram illustrating an example of the configuration of the observation support program 68. FIG. 9 is a diagram illustrating an example of control on three light emitters for each state. FIG. 10 is a diagram illustrating an example of a waveform of heart sounds.

As illustrated in FIG. 8, the observation support program 68 includes a connection module 71, a heart sounds acquisition module 72, a skin temperature acquisition module 73, an ambient temperature acquisition module 74, a heart sounds/temperature transmission module 75, an acceleration acquisition module 76, an acceleration transmission module 77, and a battery state notifying module 78. With the modules, the processing (1) to (3) as described below is executed. The modules are executed to control the blue light emitter 6511, the green light emitter 6512, and the orange light emitter 6513 as illustrated in FIG. 9.

### (1) Processing during heart sounds recording

In order to check whether the subject 10 has developed heart failure, the subject 10 has to record his/her heart sounds at predetermined intervals of time using the wearable device 2.

To start recording, the subject 10 moves the device body 20 toward the palmer side in advance as illustrated in FIG. 4. Then, the subject 10 presses the start key 654 with the hand not wearing the wearable device 2 to input a connection command. If the device body 20 is put in a sleep mode, the subject 10 first cancels the sleep mode by pressing and holding the start key 654 before inputting the connection command.

In response to the connection command input, the connection command is provided from the side key board 651 to the CPU 611. The CPU 611 then causes the connection module 71 to execute processing for connecting the wearable device 2 and the relay device 3 so that they can communicate with each other.

The CPU 611 starts blinking the green light emitter 6512 at 1/2-second intervals. In other words, the CPU 611 causes the green light emitter 6512 to be turned on for 1/4 seconds and turned off for 1/4 seconds, repeatedly. The blue light emitter 6511 and the orange light emitter 6513 are kept off (state ID = 11 in FIG. 9).

In parallel with the blinking, the CPU 611 also transmits an advertising packet via the wireless communication circuit 615. When receiving the packet, the relay device 3 transmits a connection request to the wearable device 2. In response to the connection request received by the wireless communication circuit 615, the CPU 611 establishes a connection with the relay device 3. At this time, an encryption key is used. Incidentally, in a case where the wearable device 2 has not yet been paired with the relay device 3, the CPU 611 executes processing for pairing with the relay device 3 before establishing a connection, and generates an encryption key.

Once the connection is established, the CPU 611 switches the blinking cycle of the green light emitter 6512 to 1/4-second intervals (state ID = 12). The blue light emitter 6511 and the orange light emitter 6513 are kept off.

When visually confirming that the blinking cycle of the green light emitter 6512 has changed from 1/2-second intervals to 1/4-second intervals, the subject 10 presses the device body 20 against a predetermined position of his/her chest. More specifically, in a case where the wearable device 2 is worn on the left wrist, the subject 10 presses the left palm against the central area of the anterior chest. The subject 10 then presses the switch 6514 once again to input a recording command.

In response to the recording command input, the recording command is provided from the side key board 651 to the CPU 611.

The CPU 611 then executes processing for acquiring information including heart sounds of the subject 10 using the heart sounds acquisition module 72, the skin temperature acquisition module 73, and the ambient temperature acquisition module 74. Note that the recording command and the connection command can be distinguished from each other based on whether a connection with the relay device 3 has been established. Specifically, if no connection has been established with the relay device 3, then the command from the side key board 651 is a connection command, and if a connection with the relay device 3 has been established, then the command is a recording command.

The heart sounds acquisition module 72 is used to execute the processing for acquiring heart sounds as follows. The CPU 611 turns on the microphone 621 to start sound collection. Then, an audio signal of the heart sounds collected by the microphone 621 is sampled and quantized (A/D converted), and stored in the main memory 612 or the non-volatile memory 613 as audio data.

At the time when the sound collection starts, the CPU 611 starts blinking the blue light emitter 6511 at 1/2-second intervals (state ID = 13). Incidentally, the green light emitter 6512 continues to blink at 1/4-second intervals, and the orange light emitter 6513 is kept off. After a predetermined period of time (15 seconds, for example) has elapsed since the switch 6514 was last pressed, the microphone 621 is turned off, and the audio data stored in the main memory 612 or the non-volatile memory 613 during the predetermined period of time is converted into a file. As a result, an audio file 80 is acquired which represents a waveform of heart sounds as illustrated in FIG. 10. The audio file 80 may be an uncompressed file such as a WAV format file, or a compressed file such as an MP3 file. Incidentally, it is possible to provide a separate audio processing circuit to allow the audio processing circuit to generate an audio file 80.

The skin temperature acquisition module 73 is used to execute processing for acquiring a skin surface temperature Ts of the wrist of the subject 10 as follows. The CPU 611 instructs the skin temperature measurement unit 64 to take a temperature. The skin temperature measurement unit 64 then measures the temperature at each of the three points on the wrist of the subject 10 with which the first contact 6421, the second contact 6422, and the third contact 6423 (see FIG. 6) are in contact, and provides the CPU 611 with measured values T1, T2, and T3.

In the meantime, the cephalic vein runs near the surface on the palm side of the human wrist as a major blood vessel. A portion of the skin on the palmar side of the human wrist near the cephalic vein tends to exhibit a higher temperature than the other areas because it is more susceptible to the influence of blood. Thus, in order to obtain the temperature of the skin on the wrist of the subject 10 more accurately, it is desirable to let the three contacts (first contact 6421, second contact 6422, and third contact 6423) of the skin temperature measurement unit 64 not come into contact with the portion, but rather come into contact with the other areas.

However, any one or more of the contacts possibly come into contact with the portion.

To address this, the skin temperature measurement unit 64 is so configured that when the wearable device 2 is worn on the wrist, the three contacts are aligned on a straight line in the width direction (circumferential direction) of the wrist and the distance between the centers of two adjacent contacts is approximately 8 millimeters. With this configuration, the number of contacts coming into contact with the portion can be limited to at most one.

Further, in consideration of a case where the temperature is measured with any one of the contacts coming into contact with the portion, the CPU 611 performs the following calculation.

The CPU 611 detects an abnormal value from among the three measured values T1, T2, and T3. Specifically, the measured values T1 and T2 are compared. If the difference therebetween is equal to or greater than a predetermined temperature, then the higher measured value is detected as an abnormal value. In a similar manner, the measured values T2 and T3 are compared to detect an abnormal value, and the measured value T3 and T1 are compared to detect an abnormal value.

According to this method, for example, in a case where T1 = 34.0°C, T2 = 34.6°C, T3 = 35.3°C and the predetermined temperature is 0.9°C, the measured value T3 is detected as an abnormal value. Alternatively, in a case where T1 = 34.0°C, T2 = 34.6°C, T3 = 34.7°C and the predetermined temperature is 0.9°C, no abnormal value is detected.

In a case where no abnormal value is detected, the CPU 611 calculates the average or median of the three measured values. Then, the calculated value is designated as the skin surface temperature Ts. On the other hand, in a case where an abnormal value is detected, the average of the two measured values excluding the abnormal value is calculated and the resultant is set as the skin surface temperature Ts.

Further, the ambient temperature acquisition module 74 is used to execute processing for acquiring an ambient temperature Te as follows. The CPU 611 instructs the ambient temperature measurement unit 66 to take a temperature. The ambient temperature measurement unit 66 then measures a temperature around the wearable device 2, and the measured value is provided as the ambient temperature Te from the skin temperature measurement unit 64 to the CPU 611.

The heart sounds/temperature transmission module 75 is used to execute processing for reporting the heart sounds, the skin surface temperature Ts, and the ambient temperature Te. When the skin surface temperature Ts and the ambient temperature Te are acquired, the CPU 611 generates temperature data 81 indicating the skin surface temperature Ts and the ambient temperature Te. The CPU 611 then controls the wireless communication circuit 615 to transmit the audio file 80 and the temperature data 81 to the relay device 3, and turns off the blue light emitter 6511 (state ID = 14). During the transmission, the green light emitter 6512 continues to blink at 1/4-second intervals, and the orange light emitter 6513 is kept off. After the transmission, the connection may either be maintained or terminated. In a case where the connection is terminated, all the three light emitters are tuned off.

When receiving the audio file 80 and the temperature data 81 from the wearable device 2, the relay device 3 transfers the same to the heart failure detection server 40.

While the blue light emitter 6511 blinks at 1/2-second intervals, the subject 10 is required to keep the arm wearing the wearable device 2 still. Once the blue light emitter 6511 turns off, the subject 10 is allowed to move the arm.

### (2) Processing while subject 10 is active

During the period in which the wearable device 2 is worn by the subject 10 and heart sounds are not being recorded, the three-axis accelerometer 614 measures acceleration in each of the X, Y, and Z directions at a predetermined sampling rate, and provides acceleration ax, ay, and az to the CPU 611.

The CPU 611 then uses the acceleration acquisition module 76 to temporarily store the acceleration ax, ay, and az at each time in the X, Y, and Z directions in the main memory 612 or the non-volatile memory 613 (see FIG. 7).

The CPU 611 further executes the following processing using the acceleration transmission module 77. At predetermined intervals (every hour, for example), the acceleration ax, ay, and az stored in the main memory 612 or the non-volatile memory 613 is converted into a file to thereby generate acceleration data 82. The CPU 611 then controls the wireless communication circuit 615 to transmit the acceleration data 82 to the relay device 3.

When receiving the acceleration data 82 from the wearable device 2, the relay device 3 transfers the same to the heart failure detection server 40.

Incidentally, not only when heart sounds are recorded but also when heart sounds are not recorded, the skin surface temperature Ts and the ambient temperature Te may be acquired in the method described above, and temperature data during activity 83 indicating the skin surface temperature Ts and the ambient temperature Te may be appropriately transmitted to the heart failure detection server 40 via the relay device 3.

While the acceleration is acquired, the CPU 611 keeps all of the blue light emitter 6511, the green light emitter 6512, and the orange light emitter 6513 being off (state ID = 21). However, while the skin surface temperature Ts and the ambient temperature Te are acquired together with the acceleration, only the blue light emitter 6511 is caused to blink at 1/4-second intervals (state ID = 22).

### (3) Processing for notifying charging state of secondary battery 67

In order to charge the secondary battery 67 of the wearable device 2, the subject 10 removes the wearable device 2 from the wrist and connects the USB connector 671 and a commercial power source via a USB cable. The CPU 611 then executes processing for notifying the state of the secondary battery 67 to the subject 10 based on the battery state notifying module 78 in the following manner.

When a charging control circuit detects that the USB cable is inserted into the USB connector 671 and the secondary battery 67 is being charged, the CPU 611 keeps the green light emitter 6512 turned on and forcibly turns off the blue light emitter 6511 and the orange light emitter 6513 (state ID = 01). Then, when it is detected that charging is completed, the CPU 611 forcibly turns off all of the three light emitters (state ID = 02).

After that, when the USB cable is detached from the USB connector 671, the forced light-off state is canceled. Then, as described in (1) or (2) above, the three light emitters are appropriately controlled depending on the state of the wearable device 2 and so on.

### (4. Processing by heart failure detection server 40)

When receiving the audio file 80 and the temperature data 81 (see FIG. 1), the heart failure detection server 40 executes processing for detecting heart failure. For example, the detection method described in Japanese Unexamined Patent Publication No. 2020-039472 can be used to perform the processing as follows.

The heart sounds shown in the audio file 80 (see FIG. 10) include the first heart sound and the second heart sound. Further, the third heart sound is sometimes included. The first heart sound occurs during ventricular contraction, the second heart sound occurs at the beginning of ventricular dilation, and the third heart sound is produced when the ventricular walls vibrate due to the impact of blood flow.

The heart failure detection server 40 extracts each of the above-described sounds from the heart sounds shown in the audio file 80. As a result, in a case where the third heart sound is extracted, the heart failure detection server 40 determines that heart failure has occurred, generates abnormal heart sounds notification data 84 indicating an abnormality in the heart sounds, and transmits the same to the relay device 3 or the medical personnel terminal 41. The audio file 80 and the temperature data 81 may also be transmitted to the medical personnel terminal 41.

Further, when receiving the acceleration data 82, the heart failure detection server 40 calculates an activity level of the subject 10 based on the acceleration data 82. The activity level may be calculated by using a known method. For example, the number of steps may be calculated as the activity level. The number of steps is determined by counting how many times patterns appear in the change in acceleration in the X direction during walking.

Further, the walking speed may be calculated based on the frequency of the acceleration. Alternatively, the walking speed may be classified into any one of the activity levels of "resting", "slow walking", "normal walking", "slightly brisk walking", "brisk walking", and "jogging or faster". Yet alternatively, the calorie consumption may be calculated as the activity level based on the number of steps, the walking speed, and the attributes (such as age, gender, height, weight, or muscle mass) of the subject 10.

The heart failure detection server 40 generates data indicating the activity level thus calculated, and stores the generated data in the storage as activity level data 85. In response to a request from the medical personnel terminal 41, the heart failure detection server 40 transmits the activity level data 85 thereto. Similarly, the temperature data during activity 83 is stored in the storage, and is transmitted in response to a request from the medical personnel terminal 41.

Further, the heart failure detection server 40 may determine that peripheral coldness is present when the skin surface temperature Ts is equal to or lower than a predetermined temperature.

The heart failure detection server 40 may detect heart failure also based on the peripheral coldness and the activity level. For example, in a case where the skin surface temperature Ts is equal to or lower than the predetermined temperature, the number of steps has decreased by 20% or more compared to a reference value, and the maximum walking speed has decreased by 20% or more compared to a reference value, the heart failure detection server 40 may detect that heart failure has occurred. In a case where the wearable device 2 has a function of measuring skin impedance, the heart failure detection server 40 may determine that heart failure has occurred if an additional condition of a 10% or more decrease in skin impedance is further met.

### (5. Overall processing flow and effects of the present embodiment)

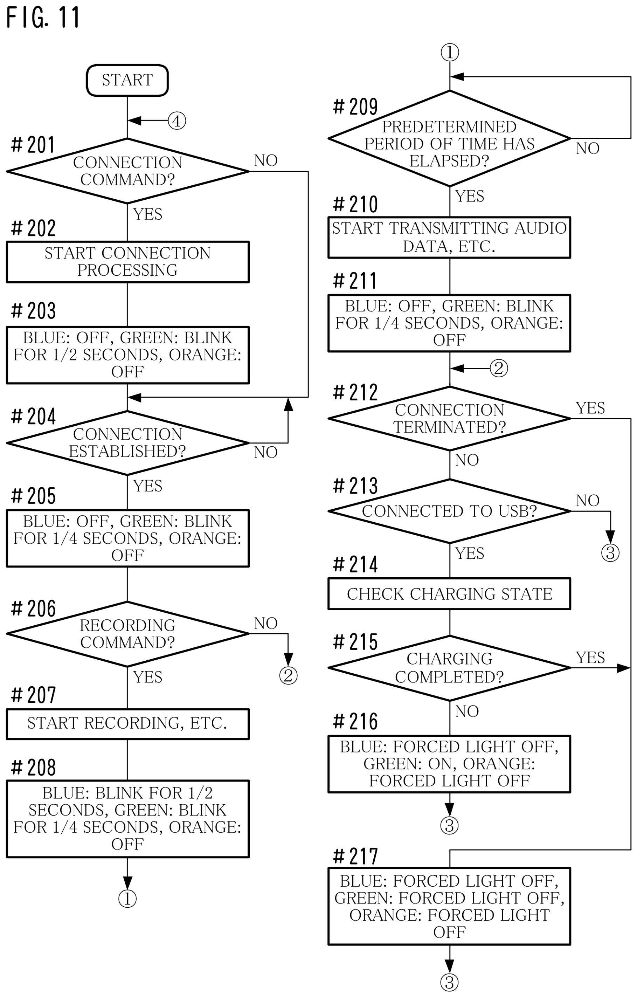

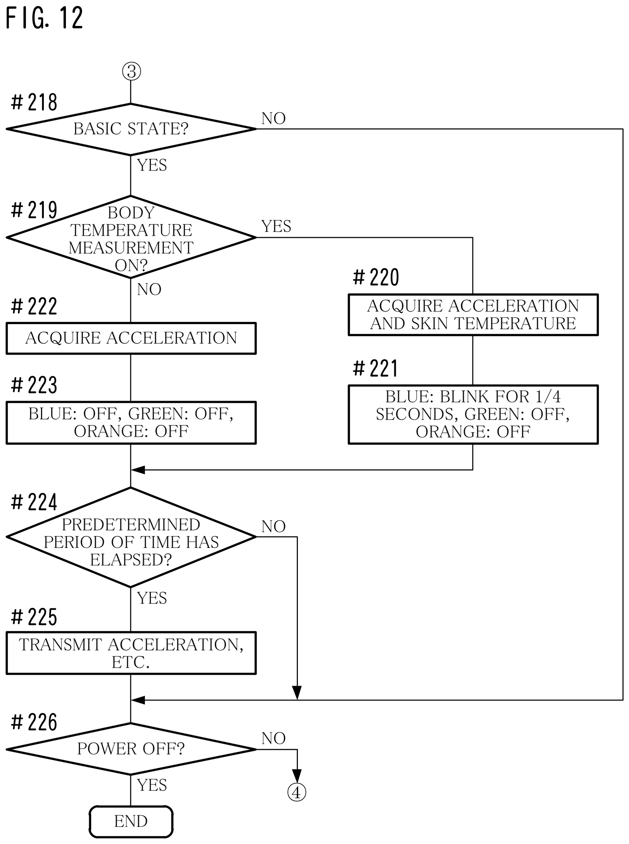

FIGS. 11 and 12 are flowcharts illustrating an example of the overall processing flow by the observation support program 68.

Next, the overall processing flow of the wearable device 2 will be described with reference to the flowcharts.

The wearable device 2 executes the processing illustrated in the steps of FIGS. 11 and 12 based on the observation support program 68.

When receiving a connection command (YES in #201 in FIG. 11), the wearable device 2 starts processing for establishing a connection with the relay device 3 (#202), turns off the blue light emitter 6511 and the orange light emitter 6513, and blinks the green light emitter 6512 at 1/2-second intervals (#203). Then, when the connection is established (YES in #204), the wearable device 2 blinks the green light emitter 6512 at 1/4-second intervals with the blue light emitter 6511 and the orange light emitter 6513 kept off (#205).

Alternatively, when receiving a recording command (YES in #206), the wearable device 2 starts recording heart sounds of the subject 10, measuring the temperature at three points on the wrist of the subject 10, and measuring the ambient temperature (#207), and further blinks the blue light emitter 6511 at 1/2-second intervals with the orange light emitter 6513 kept off and the green light emitter 6512 kept blinking at 1/4-second intervals (#208).

When a predetermined period of time (15 seconds, for example) has elapsed since the start of recording (YES in #209), the wearable device 2 stops recording, starts transmitting, as the audio file 80, data on the heart sounds recorded during the predetermined period of time to the relay device 3, starts transmitting, as the temperature data 81, data on the temperatures measured to the relay device 3 (#210), and turns off the blue light emitter 6511 with the orange light emitter 6513 kept off and the green light emitter 6512 kept blinking at 1/4-second intervals (#211).

Alternatively, when the connection is terminated (YES in #212), the wearable device 2 turns off all of the blue light emitter 6511, the green light emitter 6512, and the orange light emitter 6513 (#217).

Alternatively, while being connected to a USB cable to charge the secondary battery 67 (YES in #213), the wearable device 2 constantly checks the charging state (#214). If charging is not complete (NO in #215), then the wearable device 2 forcibly turns off the blue light emitter 6511 and the orange light emitter 6513, and keeps the green light emitter 6512 being on (#216). If charging is complete (YES in #215), then the wearable device 2 forcibly turns off all of the three light emitters (#217).

If the wearable device 2 is in a basic state where neither heart sounds collection nor charging of the secondary battery 67 is being performed (YES in #218 in FIG. 12), then the wearable device 2 acquires acceleration and so on, as follows.

If body temperature measurement is set to on (YES in #219), then the wearable device 2 measures the acceleration in each of the X, Y, and Z directions, and also measures the temperature at each of the three points on the wrist of the subject 10 (#220). During measurement, in principle, the blue light emitter 6511 is caused to blink at 1/4-second intervals, and the green light emitter 6512 and the orange light emitter 6513 are turned off (#221).

On the other hand, if the body temperature measurement is set to off (NO in #219), then the wearable device 2 measures the acceleration in each of the X, Y, and Z directions (#222). It is noted that temperature is not measured. During measurement, basically, all of the three light emitters are kept off (#223).

Each time a predetermined period of time (1 hour, for example) has elapsed (YES in #224), the wearable device 2 transmits, to the relay device 3, data indicating the acceleration or temperature acquired in Step #220 or #222 as the acceleration data 82 or the temperature data during activity 83 (#225). In a case where a connection with the relay device 3 has not yet been established, a connection is established. During establishment, the green light emitter 6512 may be caused to blink at 1/2-second intervals, and during transmission, the green light emitter 6512 may be caused to blink at 1/4-second intervals.

The wearable device 2 executes the processing of Steps #201 to #225 appropriately until the power is turned off.

According to the present embodiment, the condition of a subject's heart can be monitored in a less invasive manner and at lower cost than conventional methods. This enables medical personnel to intervene early to treat the subject 10 before the symptoms of heart failure worsen, thereby reducing hospital readmissions associated with high medical costs compared to conventional approaches. Further, the skin surface temperature Ts is calculated after excluding an abnormal value, which allows for a more accurate assessment of peripheral coldness than is conventionally possible.

Moreover, the subject 10 can acquire heart sounds more easily than with conventional configurations. Specifically, the subject 10 wears the wearable device 2 so that the device body 20 is, for example, on the palmer side of the subject's left wrist. Thereby, when the left arm is bent and the left palm is pressed against the central area of the anterior chest, the microphone 621 is pressed against the optimal position directly above or near the heart of the subject 10. Accordingly, more accurate heart sounds can be obtained with high sensitivity and low noise. At this time, the position of the device body 20 with respect to the left hand is constant. Accordingly, by remembering the position when the left arm is bent and the left palm is pressed against the chest, the microphone can be always pressed against the correct position to obtain heart sounds in the same condition, heart sounds can be analyzed under the same condition, which increases the accuracy of detection of heart failure. Further, since heart sounds can be obtained by a simple operation of pressing the start key 654 with the left arm bent and the left palm pressed against the chest, heart sounds can be reliably obtained on a daily and regular basis without imposing burden on the subject 10.

In addition, the operation unit with which the subject 10 performs operation for obtaining heart sounds, namely, the user interface unit 65, is disposed on the side surface 204 of the device body 20 aligned with the longitudinal direction of the arm of the subject 10 when worn on the wrist. Accordingly, when the subject 10 bends his/her left arm to press the microphone against his/her chest, the user interface unit 65 is positioned on the upper surface and is easily visible to the subject 10, which makes it extremely easy to operate the user interface unit 65 to obtain heart sounds.

Further, the progress of the process for acquiring heart sounds can be easily seen with light of LEDs such as the blue light emitter 6511. Thus, the subject can maintain the posture required for heart sounds acquisition in a comfortable and reassuring manner. During heart sounds acquisition, the likelihood of the microphone shifting off or moving away from the chest due to arm movements is reduced. As described above, the microphone is placed near the optimal position for detecting the third heart sound, which is useful for heart failure detection, so that the patient can easily and independently acquire and record heart sounds at home with high reproducibility.

These effects are clearly more significant and advantageous than those of the technologies described in Patent Literatures 1 to 3. The device described in Patent Literature 1 cannot be worn continuously by a subject and, thus, regular daily acquisition of heart sounds is often neglected. The heart rate monitor described in Patent Literature 2 is usually concealed beneath clothing, which makes it difficult for a subject to confirm the state and to operate the heart rate monitor. The heart rate monitor described in Patent Literature 3 is not designed for easy operation by a subject during measurement. In contrast, the wearable device 2 according to the present embodiment can solve these problems.

### (6. Modification)

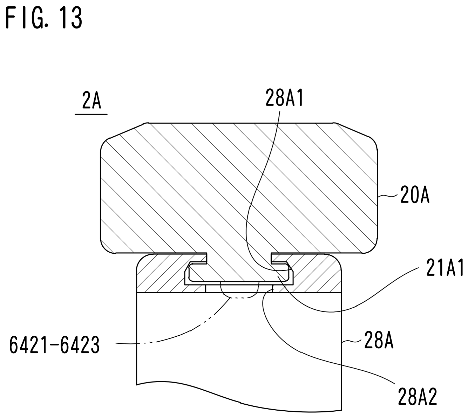

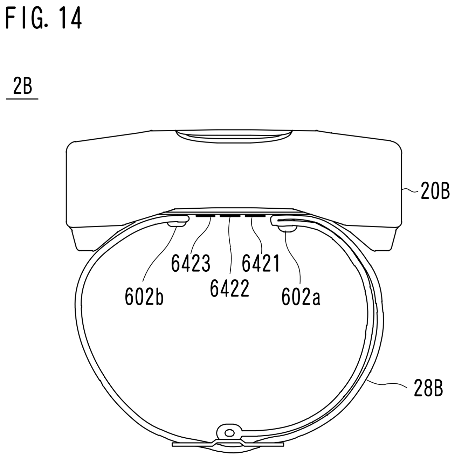

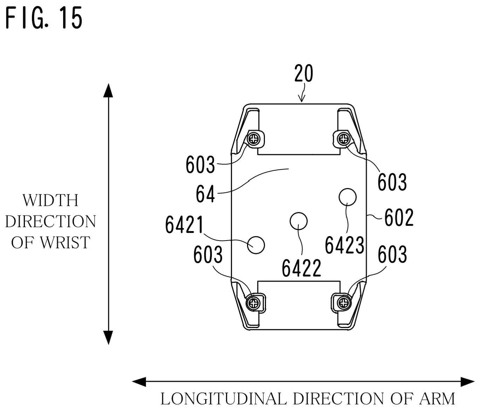

FIG. 13 is a diagram illustrating a first modification to the wearable device 2. FIG. 14 is a diagram illustrating a second modification to the wearable device 2. FIG. 15 is a diagram illustrating a modification to the arrangement of three contacts.

In the present embodiment, the subject 10 moves the entire wearable device 2 in order to record heart sounds. However, the wearable device 2 may be so configured that only the device body 20 can be moved, for example, as illustrated in FIG. 14.

A wearable device 2A illustrated in FIG. 14 is the first modification to the wearable device 2. In the wearable device 2A, a protrusion 21A1 of a device body 20A fits into and engages with a groove 28A1 provided on an outer periphery of a wristband 28A, and the device body 20A can slide around the top or the bottom of the wrist along the outer periphery of the wristband 28A, and rotate about 180 degrees.

The wristband 28A is worn on the wrist with a known fastener such as that illustrated in FIG. 2 or not illustrated. The wristband 28A does not rotate with respect to the wrist by itself.

The groove 28A1 is provided at the longitudinal center of the wristband 28A and extends for approximately half the circumference of the wrist. At each end of the groove 28A1, a cutout 28A2, measuring approximately 1 to 2 centimeters in length, is provided at the base of the groove 28A1.

In the protrusion 21A1, three contacts (first contact 6421, second contact 6422, and third contact 6423) are arranged so as to be biased outward by a spring having good thermal conductivity or the like. When the protrusion 21A1 slides inside the groove 28A1, ends of the three contacts are received in contact with the base of the groove 28A1. When the protrusion 21A1 is positioned on the ends of the groove 28A1, the three contacts come into direct contact with the skin surface of the wrist via the cutouts 28A2.

Alternatively, the wearable device 2 may be configured as illustrated in FIG. 14. A wearable device 2B illustrated in FIG. 14 is the second modification to the wearable device 2. In the wearable device 2B, both ends of a wristband 28B are coupled not to ends of a device body 20B but coupled at positions 602a and 602b with the three contacts at the center (first contact 6421, second contact 6422, and third contact 6423) interposed therebetween.

Accordingly, when the wearable device 2B is worn on the wrist, the distance between both ends of the wristband 28B is short, which allows the flexible wristband 28B to rotate around the wrist smoothly without obstruction by the entire length of the device body 20B.

In the present embodiment, the activity level of the subject 10 is calculated by the heart failure detection server 40, but it may be calculated by the wearable device 2 or the relay device 3. Further, in the present embodiment, the processing for detecting heart failure is executed by the heart failure detection server 40, but it may be executed by the wearable device 2 or the relay device 3.

In the present embodiment, the skin surface temperature Ts is calculated by the wearable device 2, but it may be calculated by the relay device 3 or the heart failure detection server 40.

The wearable device 2 may be equipped with the functions of a smart watch, for example, as follows. Transparent rubber such as highly transparent silicone rubber is used as the rubber 623 (see FIG. 5), and a transparent piezoelectric film is used as the microphone 621. Then, an organic EL display is placed on the back surface of the microphone 621. The organic EL display is controlled by the CPU 611, and displays the date, time, the state of the wearable device 2, and the ambient temperature Te, as well as the result of heart failure detection, the activity level, the skin surface temperature Ts, and so on of the subject 10, as necessary. Further, a touch panel may be provided between the microphone 621 and the organic EL display.

In the present embodiment, the state of the wearable device 2 is notified to the subject 10 by illuminating the three light emitters, but it may be notified by outputting sound from the speaker 63. Another configuration is possible in which the time for heart sounds acquisition is set using a clock function and the speaker 63 outputs an alarm each time the set time is reached. This allows heart sounds to be acquired at a precise time and prevents missed acquisition of heart sounds.

In the present embodiment, the three contacts (first contact 6421, second contact 6422, and third contact 6423) are located on a straight line; however, the three contacts may be located on a curve corresponding to the circumference of the wrist.

In the present embodiment, the three contacts are provided on a straight line perpendicular to the longitudinal direction of the arm. However, the invention is not limited thereto as long as the distance between any two of the contacts in the width direction of the wrist is equal to or greater than a predetermined distance. If this condition is satisfied, the three contacts may be disposed as illustrated in FIG. 15 so that the positions in the longitudinal direction of the arm are different from each other, for example. The predetermined distance range is set based on a route of cephalic vein near the wrist. Specifically, setting is so made that when any one of the three contacts is positioned directly above the cephalic vein, the other two contacts are positioned at a predetermined distance or more from directly above the cephalic vein.

If the contacts are arranged in this manner, the number of contacts is not limited to three. The number of contacts may be, for example, two, or four or more. In either case, the wearable device 2 compares any two measured values, and if a difference therebetween is a predetermined temperature or higher, then the higher measured value is detected as an abnormal value. The wearable device 2 then calculates, as the skin surface temperature Ts, the average or median of the measured values other than the abnormal value.

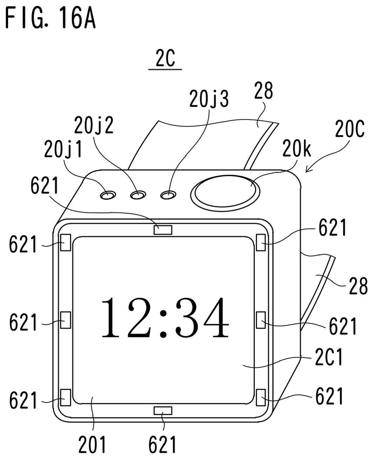

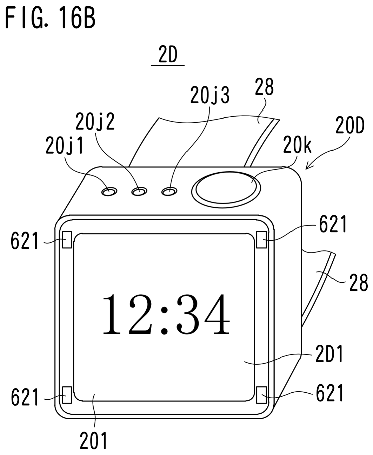

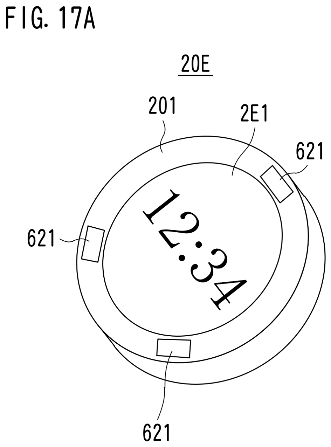

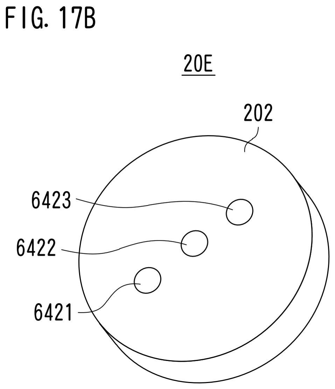

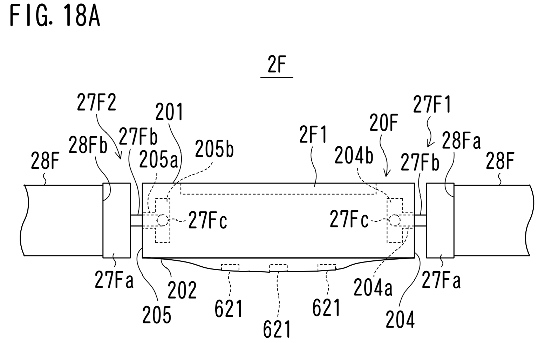

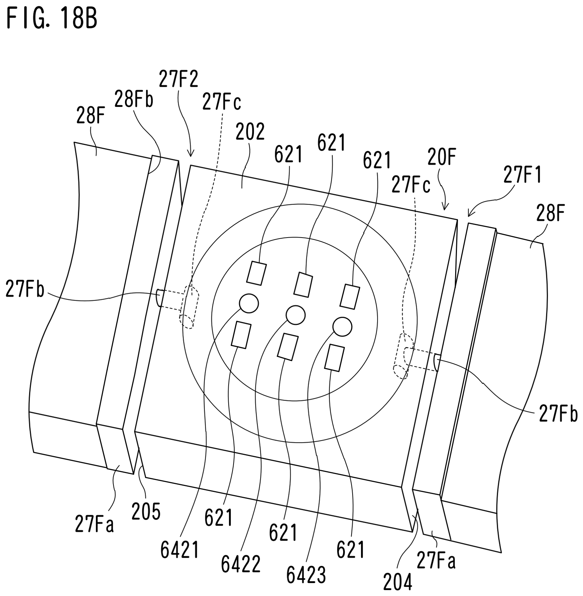

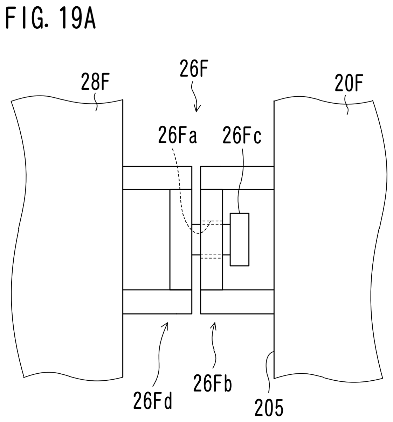

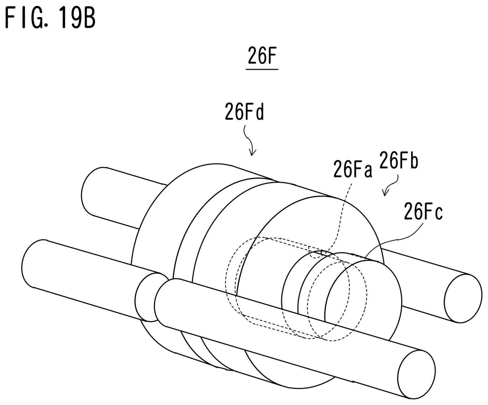

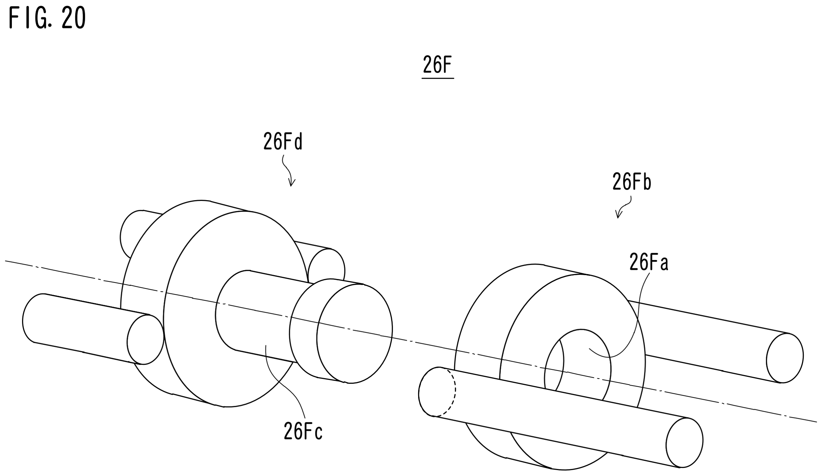

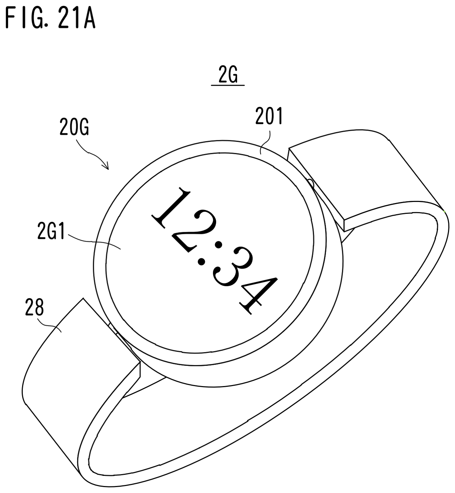

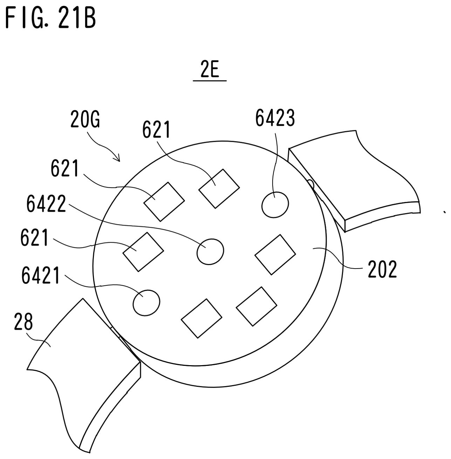

FIG. 16 is a diagram illustrating an example of the appearance of each of wearable devices 2C and 2D. FIG. 17 is a diagram illustrating an example of the appearance of an upper surface side and a lower surface side of a device body 20E. FIG. 18 is a diagram illustrating an example of the appearance of a side surface side and a lower surface side of a wearable device 2F. FIG. 19 is a front view and a perspective view of a coupling member 26F. FIG. 20 is a diagram illustrating an example of a female part 26Fb and a male part 26Fd. FIG. 21 is a diagram illustrating an example of the appearance of each of an upper surface side and a lower surface side of a wearable device 2G.

In the present embodiment, one circular piezoelectric element with a diameter of 20 millimeters or so is used as the microphone 621. Instead, one or more microphones employing micro electro mechanical systems (MEMS) on the order of a few millimeters by a few millimeters (hereinafter, referred to as "MEM microphones") may be used. A MEMS microphone is a small microphone in which a MEMS chip for converting sound into an electrical signal and an IC chip for signal processing are assembled and packaged together. The IC chip may be provided with functions such as a filter function for passing or blocking a specific frequency, a noise removal function, and an automatic level adjustment function. Alternatively, a condenser microphone (ECM) may be used as the microphone 621. Further, the wearable device 2 may be provided with a display on the upper surface thereof.

For example, as in the wearable device 2C illustrated in FIG. 16(A), an organic EL display may be disposed, as a display 2C1, at the center of an upper surface 201 of a device body 20C, and eight MEMS microphones may be disposed therearound instead of the microphone 621. Further, a thin rubber film such as silicone may be attached to the surface of the microphone 621 for dust-proof or water-proof.

Further, in the wearable device 2C or the relay device 3, noise cancellation may be applied to sounds collected by the plurality of microphones 621. For example, noise components may be removed and heart sound waveforms may be smoothed by comparing or compensating phases of audio signals from the microphones 621. This enables high-quality heart sounds recording with reduced ambient noise. Incidentally, as with the device body 20 (see FIG. 6), the first contact 6421, the second contact 6422, and the third contact 6423 are disposed on the lower surface (back surface) 202 to measure the skin temperature (not illustrated).

Alternatively, as in the wearable device 2D illustrated in FIG. 16(B), six MEMS microphones are disposed, instead of the microphone 621, on an upper surface 201 of a device body 20D, and a display 2D1 may be also disposed. Alternatively, as illustrated in FIGS. 17(A) and 17(B), a thick and disk-shaped casing may be used as a casing of a device body 20E, an organic EL display having a circular display surface may be disposed as a display 2E1, and a plurality of (three, for example) MEMS microphones may be disposed therearound instead of the microphone 621.

Alternatively, as in the wearable device 2F illustrated in FIGS. 18(A) and 18(B), together with the first contact 6421, the second contact 6422, and the third contact 6423 of the skin temperature measurement unit, a plurality of (six, for example) MEMS microphones may be disposed on a lower surface 202 as the microphones 621, and an organic EL display may be disposed on an upper surface 201 as a display 2F1.

Further, the wearable device 2F may be so configured that a device body 20F is rotatable around an axis corresponding to the longitudinal direction of an elastic wristband 28F to thereby eliminate the need to remove the wearable device 2F from the arm when recording heart sounds. For example, the wearable device 2F may be configured as follows.

Cylindrical holes 204a and 205a are provided on two side surfaces 204 and 205 of the device body 20F respectively perpendicular to the longitudinal direction of the wristband 28F, i.e., the circumferential direction of the wrist. Further, disk-shaped spaces 204b and 205b with diameters larger than those of the holes 204a and 205a are concentrically provided immediately beyond the holes 204a and 205a, respectively. The wearable device 2F includes two metal coupling members 27F1 and 27F2.

The coupling member 27F1 is configured such that a base portion 27Fa, a shaft portion 27Fb, and an anchoring portion 27Fc are integrally formed. The base portion 27Fa is fixed to one end 28Fa of the wristband 28F. The shaft portion 27Fb passes through the hole 204a, the anchoring portion 27Fc is rotatably disposed in the space 204b, and serves as a retainer to prevent the shaft portion 27Fb from coming out of the hole 204a. The anchoring portion 27Fc can be implemented by, for example, a pin biased radially outward by a spring relative to the shaft portion 27Fb. Alternatively, the space 204b can be made as a part of an inner space of the casing of the device body 20F, and the anchoring portion 27Fc can be integrated with the shaft portion 27Fb by a screw or welding at the time of assembling the device body 20F. The diameter of the shaft portion 27Fb is slightly smaller than the diameter of the hole 204a, and a clearance of about 0.1 millimeters is formed therebetween. The maximum length between both end faces of the anchoring portion 27Fc is greater than the diameter of the hole 204a, which allows the anchoring portion 27Fc to abut against the wall surface of the space 204b to prevent it from coming loose. In a case where the shaft portion 27Fb and the anchoring portion 27Fc are integrated, the entire length of the anchoring portion 27Fc can be made greater than the diameter of the hole 204a, for example, by 3 millimeters or so.

As described above, the coupling member 27F1 couples the side surface 204 of the device body 20F and the end 28Fa of the wristband 28F so that the device body 20F and the wristband 28F rotate relatively with the shaft portion 27Fb as the axis of rotation. As with the coupling member 27F1, the coupling member 27F2 couples the side surface 205 of the device body 20F to the other end 28Fb of the wristband 28F.

When heart sounds are not recorded, the subject 10 wears the wearable device 2F so that the lower surface 202 of the device body 20F is in contact with the arm of the subject 10. On the other hand, when heart sounds are recorded, the device body 20F is moved appropriately toward the palmer side, and the device body 20F is rotated by 180 degrees so that the lower surface 202 is visible. The subject 10 then places the lower surface 202 near his/her heart to record heart sounds.

In the meantime, with the wearable device 2, when heart sounds are not recorded, not only the skin temperature of the subject 10 but also information on the pulse (for example, pulse wave, pulse rate) can be measured. In short, information on the pulse can be obtained all the time. Further, when heart sounds are recorded, the temperature near the heart, i.e., the core body temperature or central temperature can be measured. Using such information allows for more precise heart failure diagnosis or early detection of indicators thereof.

Instead of the coupling member 27F, the coupling member 26F as illustrated in FIGS. 19(A) and 19(B) may be used. The coupling member 26F is a metal component that is often used in products such as key chains, and includes a female part 26Fb having a cylindrical hole 26Fa and a male part 26Fd having a headed cylindrical pin 26Fc as illustrated in FIG. 20. The cylindrical part of the pin 26Fc passes through the hole 26Fa, and the female part 26Fb and the male part 26Fd can relatively rotate with the pin 26Fc as an axis of rotation. One end of the female part 26Fb and the male part 26Fd is fixed to the device body 20F, and the other end is fixed to the wristband 28F. This allows the device body 20F to rotate with respect to the wristband 28F.

Although the user interface unit 65 (see FIG. 2) is omitted in FIG. 18, it is desirable to place the user interface unit 65 between the coupling member 27F (or the coupling member 26F) and the upper surface 201 on the side surface 204 of the device body 20F so as to avoid the coupling member 27F or a part connected to the coupling member 27F, namely, the axis of rotation. Alternatively, the user interface unit 65 may be disposed on another side surface such as the side surfaces 203 and 205 (see FIG. 6). Yet alternatively, the user interface unit 65 may be provided on the surface of the wristband 28F in the vicinity of the end to be coupled to the side surface 204.

In a case where the microphone 621 is disposed on the lower surface, a thick and disk-shaped casing is used, as in the wearable device 2G illustrated in FIG. 20, a circular organic EL display may be disposed as a display 2G1, and a plurality of (for example, three) MEMS microphones may be disposed therearound as the microphone 621.

The configuration of the whole or each part of the heart failure detection system 1, the wearable devices 2, and 2B to 2G, the content of the processing, the order of the processing, etc. may be modified as appropriate in accordance with the spirit of the present invention.

### Reference Signs List

1: Heart failure detection system
10: Subject
40: Heart failure detection server (heart failure detection device)
2, 2B to 2G: Wearable device
20, 20B to 20G: Device body (body)
201: Upper surface
202: Lower surface
204: Side surface (operation surface)
28, 28F: Wristband
611: CPU (calculation means)
614: Three-axis accelerometer
62: Sound collection portion
621: Microphone
623: Rubber
65: User interface unit (operation portion)
6511: Blue light emitter (display means)
6512: Green light emitter (display means)
6513: Orange light emitter (display means)
6421: First contact (contact)
6422: Second contact (contact)
6423: Third contact (contact)
654: Start key (key)
66: Ambient temperature measurement unit (ambient temperature measurement means)
73: Skin temperature acquisition module (calculation means)

## Claims

1. A wearable device that is worn on a wrist of a subject and used, comprising:
a sound collection portion that is provided on an upper surface or a lower surface of a body of the wearable device and is configured to acquire heart sounds of the subject; and
an operation portion that is provided on a side surface of the body along a longitudinal direction of an arm of the subject when the wearable device is worn on the wrist and is configured to perform, by the subject, operation for acquiring the heart sounds.

2. The wearable device according to claim 1, wherein
the sound collection portion includes rubber and a microphone having a piezoelectric element, and
a surface of the rubber forms a part of the upper surface, and the microphone is disposed in contact with a back surface of the rubber.

3. The wearable device according to claim 2, wherein
a display is provided on a back surface of the microphone, and
the rubber is transparent rubber, and the microphone is a transparent piezoelectric element.

4. The wearable device according to claim 1, wherein
a display is provided on the upper surface,
the sound collection portion includes one or more micro electro mechanical systems (MEMS), and
said one or more MEMS are disposed on the upper surface together with the display.

5. The wearable device according to any one of claims 1 to 4, comprising
an elastic wristband that is coupled to the body and is configured to be wrapped around the wrist to allow the body to be worn on the wrist, wherein
the body is attached to a dorsal side of the subject when the heart sounds are not acquired, and the body is attached to a palmer side of the subject when the heart sounds are acquired.

6. The wearable device according to claim 1, wherein
a display is provided on the upper surface,
the sound collection portion includes one or more micro electro mechanical systems (MEMS), and
said one or more MEMS are disposed on the lower surface.

7. The wearable device according to claim 6, comprising
an elastic wristband configured to attach the body to the wrist, wherein
the wristband is so coupled to the body as to rotate with an axis in a longitudinal direction of the wristband used as an axis of rotation, and
the body is attached to a dorsal side or a palmer side of the wrist to allow a contact of the lower surface when the heart sounds are not acquired, and the body is attached to the palmer side of the wrist to allow a contact of the upper surface when the heart sounds are acquired.

8. The wearable device according to any one of claims 1 to 7, wherein
the operation portion includes a key with which the subject inputs an instruction, and a display means configured to notify the subject of a state of the wearable device.

9. The wearable device according to claim 8, comprising
a communication means configured to perform communication with another device, and
a control means configured to, in collecting the heart sounds, control the communication means so that the wearable device and said another device are connected to each other when a connection instruction is input via the key as the instruction, and configured to, when the wearable device and said another device are connected and then a recording instruction is input via the key as the instruction, control the collection portion to acquire the heart sounds.

10. The wearable device according to any one of claims 1 to 9, wherein
in order to measure temperatures at a plurality of locations on the wrist, a plurality of contacts is disposed on a lower surface of the body such that when the wearable device is worn, a distance between two adjacent contacts of the plurality of contacts in a width direction of the wrist falls within a predetermined range, and
the wearable device includes a calculation means configured to calculate a temperature of the wrist based on the temperatures at the plurality of locations detected by the plurality of contacts.

11. The wearable device according to claim 10, wherein
the plurality of contacts is three contacts, and
the calculation means compares between two measured values of three measured values detected by the three contacts, and if a difference between the two measured values is equal to or greater than a predetermined temperature, detects the higher measured value as an abnormal value, and calculates, as the temperature of the wrist, a representative value of the measured values other than the abnormal value of the three measured values.

12. The wearable device according to claim 11, wherein
the three contacts are so set that, when any one of the contacts is located at a position directly above ulnar artery or at a position directly above radial artery, the other two contacts are located at a certain distance or more from both of the two positions.

13. The wearable device according to any one of claims 1 to 12, comprising
an ambient temperature measurement means configured to measure an ambient temperature.

14. The wearable device according to any one of claims 1 to 13, comprising
a three-axis accelerometer configured to calculate an activity level of the subject.

15. A wearable device that is worn on a wrist of a subject and used, comprising:
an elastic wristband configured to attach a body of the wearable device to the wrist;
a sound collection portion that is provided on a lower surface of the body and is configured to acquire heart sounds of the subject; and
a display provided on an upper surface of the body, wherein
the wristband is so coupled to the body as to rotate an axis in a longitudinal direction of the wristband as an axis of rotation.

16. A wearable device that is worn on a wrist of a subject and used, wherein
in order to measure temperatures at a plurality of locations on the wrist, a plurality of contacts is disposed on a lower surface such that when the wearable device is worn, a distance between two adjacent contacts of the plurality of contacts in a width direction of the wrist falls within a predetermined range.

17. A heart failure detection system comprising:
the wearable device according to any one of claims 1 to 16;
and
a heart failure detection device configured to detect heart failure of the subject based on the heart sounds acquired by the wearable device.
